# EUROPEAN PATENT SPECIFICATION

(11) **EP 2 414 021 B1**
(45) Date of publication and mention of the grant of the patent: **13.03.2013**
(21) Application number: 10723269.6
(22) Date of filing: 30.03.2010
(51) Int. Cl.: A61M 25/09

(54) **GUIDEWIRE WITH ANCHOR**
FÜHRUNGSDRAHT MIT ANKER
FIL GUIDE AVEC ANCRE

(30) Priority: 31.03.2009 GB 0905554
(43) Date of publication of application: 08.02.2012
(73) Proprietor: Royal Brompton and Harefield NHS Foundation Trust, London SW3 6NP (GB)
(72) Inventor: MULLEN, Michael, London SW3 6NP (GB)
(74) Representative: Forsythe, Dominic
(86) International application number: PCT/GB2010/000617
(87) International publication number: WO 2010/112837

(56) References cited:
- US-A1- 2005 113 862
- US-A1- 2005 273 147
- US-A1- 2006 259 063

## Description

The present invention relates to a guidewire for a catheter, a deployment system for a guidewire and a method of using the guidewire and deployment system. In particular, the present invention relates to a guidewire having an anchoring mechanism for controlling and/or limiting longitudinal displacement of the guidewire when fully deployed.

Guidewires are commonly used to facilitate access to cavities within the body such as the cardiovascular, gastrointestinal and urological systems. Guidewires provide a first route to the site of interest, and delivery systems can be passed over the wire to reach the site with a minimum level of trauma and without loss of position.

Interventional cardiology guidewires are used within the vascular lumens of the patient to access the heart. Wires are generally introduced into the femoral artery or vein at the groin and fed up to the left or right side of the heart.

For example, coronary guidewires may be introduced into the femoral artery and passed into the coronary arteries of the heart to allow a balloon system to be delivered to open a narrowed artery. Alternatively, electrophysiology catheters can be passed over guidewires in the femoral vein and right atrium to detect and treat electrical disturbances of the heart. These techniques have been in use for over 15 years now and are well established wire guided techniques.

More recently, structural interventions, including valve replacement, have been performed percutaneously. This imposes a greater demand on the guidewire to maintain position whilst the procedure is being carried out. As the distal end of the wire is effectively free floating within the blood vessel, the user has little control over its position.

During procedures such as valve replacement, a great deal of manipulation is required to locate the delivery system at the site of interest. Tortuous anatomy and small bend radii can put stress on the delivery catheter and wire. In order to facilitate pushing of the delivery catheter over the guidewire, it may be necessary to apply significant pushing and pulling forces to the wire and catheter relative to each other, for example to partially straighten out portions of the delivery catheter. As the guidewire end is free, this can be difficult to achieve.

For example, pulmonary valve placement requires the delivery catheter and wire to cope with the anatomy of the femoral vein, the curvature of the right atrium and the 180 degree rotation between entering the tricuspid valve and crossing the pulmonary valve, all within the confines of the right ventricle.

Correct valve placement is critical whether in the pulmonary position or the aortic site. If the valve is incorrectly aligned, valve insufficiency or paravalvular leak may occur.

Also, the guidewire needs to be stiff enough to control the delivery system. This stiffness increases the risk of damage to the patient, such as pulmonary artery puncture. The guide wires may be provided with a softer distal tip to reduce this risk. However, the possibility of a fatal haemothorax remains.

US 2006/0259063 A1 discloses wire guide devices having distal anchoring devices. One device includes a wire guide having a distal portion operatively coupled to a holding body having a self-expanding end portion having wire members capable of assuming a first radially compressed configuration and a second radially expanded resilient configuration. Another device includes a wire guide distal portion operatively coupled to a distal anchoring device having a distal self-expanding suspension portion having a plurality of stabilizers capable of assuming a first radially compressed configuration when constrained by the outer sheath and a second radially expanded resilient configuration when the sheath is withdrawn proximally. An elongate outer sheath with first and second openings defining a lumen therebetween and slideably constrain the self-expanding portions to the compressed configuration. Expanded configurations help keep the wire guide distal portion at a target site within a body lumen.

US 2005/0273147 A1 discloses a guide wire for a stent assembly, the guide wire including an outwardly expandable and inwardly contractible deformable portion.

US 2005/0113862 A1 discloses a self-activating endoluminal device. The device includes at least a control element (such as a guide wire) connected to a surrounding sheath and an elastic bias section to control changes to a bias force formed between the control element and the sheath. By applying an external force at a proximal end of the device, the shape can change between varying degrees of deformed shapes and an undeformed shape. In this way, both ease of insertion into the body lumen and anchoring to the lumen is promoted. A distal end of the assembly can be made to change shape for improved steerability, anchoring or both. In a particular form, the anchoring section can work as a floating parachute-like device to pull the assembly by means of the flow in the body lumen, while in a more particular form, the floating parachute-like device may be modified to act as a filter for trapping emboli.

It is an object of the present invention to provide apparatus and methods to overcome at least one of the problems discussed above in relation to the prior art.

According to an aspect of the invention, there is provided a guidewire for a catheter according to claim 1.

The closed path could comprise a closed loop spanning the axis of a lumen forming part of the body cavity, duct or vessel. In other words, the closed path could follow a generally circumferential locus around the lumen, surrounding the lumen, optionally with some circumferentially local structure (e.g. "zig-zagging") to improve grip. In this case, the "axis" of the closed path or loop could be said to be parallel to the longitudinal axis of the lumen in the region where the anchor is deployed. Alternatively, the closed path or loop could be a closed cell pressed against a local region of the side wall of the lumen. In this case, an axis of the closed path or loop could be said to be aligned so as to be substantially perpendicular to the longitudinal axis of the lumen in the region of the anchor (a plane of the loop is substantially parallel to the portion of the wall of the lumen with which it is in contact). In any event, the provision of a closed-path line of contact improves the gripping function of the anchor by the fact that, when the portion of the engaging portion corresponding to the closed path is pressed against the wall of the lumen, tissue will tend to protrude radially inwards either side of the contact line (including a portion where the protusion will be substantially in front of and behind the contact line along an axial direction relative to the lumen). This protrusion of tissue inhibits longitudinal movement of the anchor because the tissue resists the deformation necessary to allow the anchor to move (thus requiring different portions of tissue to protude). This effect is in addition to the normal frictional forces that will also occur between the closed loop line of contact and the portion of the tissue immediately radially outside thereof.

Providing an efficient anchor on the guidewire to inhibit longitudinal movement of the guidewire once deployed helps to ensure that the guidewire maintains its position within the body cavity, duct or vessel while subsequent procedures are carried out. For example, the anchor allows a slight longitudinal pulling force to be applied to the guidewire, which can be used to straighten the guidewire and/or delivery catheters fed over the guidewire. Delivery catheters can thus be pushed over the guidewire more easily and with a reduced risk to the patient in comparison with the situation when the guidewire end is free or unreliably anchored. This may be particularly important in procedures such as valve replacement, as discussed above, where a great deal of manipulation is required to locate the delivery system at the site of interest, and navigation of torturous anatomy and small bend radii is required.

More generally, the improved control over the position of the guidewire provided by the present invention facilitates control of the positioning of components delivered to a treatment site using the guidewire. This is of critical importance in many applications. For example, in valve replacement procedures, as discussed above, correct valve placement is critical whether in the pulmonary position or the aortic site. If the valve is incorrectly aligned, valve insufficiency or paravalvular leak may occur.

Optionally, the guidewire is formed from a single elongated wire, with the anchor formed by shaping a distal end portion of the wire. For example, the distal end of the wire may be shaped so as to press radially against the walls of the body cavity, duct or vessel within which it is deployed in order to provide a gripping force. Such arrangements are advantageous because they can be manufactured cheaply and the absence of joins and/or other such structural complexities, provides for improved reliability and also naturally tends to minimise disruption of fluid flow within the body cavity, duct or vessel, which may be advantageous in many applications.

Optionally, the anchor can be reversibly switched between a radially constrained state suitable for insertion of the guidewire and a radially expanded state suitable for providing the anchoring force. Preferably, the switch can be performed a plurality of times. This functionality facilitates removal and/or repositioning of the anchor.

Preferably, where an engaging portion making a closed-path contact line is provided, an average angle between elements of the closed path and a direction parallel to the longitudinal axis of the anchor is larger in the radially expanded state than in the radially constrained state. This feature conveniently enhances the grip in the radially expanded state, while reducing the grip in the radially constrained state.

The anchor may comprise an engaging portion configured to engage with the body cavity, duct or vessel and a connecting portion for connecting the engaging portion to a stem of the guidewire, wherein the connecting portion is configured such that longitudinal advancement of a delivery catheter can engage with said connecting portion and cause switching of the anchor between the radially expanded state and the radially constrained state.

Optionally, the engaging portion comprises a collapsible frame.

The connecting portion may comprise a plurality of rods obliquely angled relative to the axis of the anchor and diverging from a root thereof to a plurality of separated points of connection with the engaging portion.

Optionally, the anchor is formed so as to disrupt the flow of fluid within the body cavity, duct or vessel to a minimum extent. For example, in the region of the anchor, the flow impedance is preferably increased by no more than 5 per cent in comparison with the flow impedance in the same region when the anchor is not present. More preferably, the flow impedance is increased by no more than 2 per cent, 1 per cent, 0.1 per cent or 0.01 per cent.

Optionally, the anchor is formed so as to exert a force radially on the walls of the body cavity, duct or vessel that increases as a function of a pulling force applied by a user in a longitudinal direction of the guidewire. This arrangement allows the gripping force to increase in proportion to the force applied by a user. Thus, at least for forces up to a given threshold, the anchoring force provided by the anchor can be such as to balance an applied force (so that the guidewire does not move longitudinally) while minimising stress in the body cavity, duct or vessel caused by the presence of the anchor. In other words, rather than arranging for the anchor always to exert a radial force that is sufficient to sustain the maximum pulling force that the anchor is able to balance (i.e. a pulling force equal to the threshold force), the anchor of the present invention provides a variable radial force, allowing smaller radial forces where only a relatively small anchoring force is required.

For applied longitudinal forces above the threshold, the anchor may be configured simply to collapse, which facilitates removal of the guidewire without the need for separate apparatus. Manufacturing costs may therefore be reduced and it may also be advantageous to reduce the complexity and/or number of different components that need to be inserted into the patient via the guidewire. Alternatively, a removal catheter may be used to assist with removal of the guidewire after use.

According to a further aspect of the invention, there may be provided a system for performing interventional cardiology, comprising: a guidewire according to an embodiment of the invention; and a delivery catheter for delivering components for carrying out cardiology treatment or diagnosis.

According to a further aspect of the invention, there is provided a deployment system, comprising: a guidewire according to an embodiment of the invention; and a guidewire delivery catheter for delivering said guidewire to a desired location within said body cavity, duct or vessel, said guidewire delivery catheter comprising means for actuating said anchor.

Embodiments of the invention will now be described, by way of example only, with reference to the accompanying schematic drawings in which corresponding reference symbols indicate corresponding parts, and in which:
Figure 1A shows a guidewire with a radially constrained anchor within a guidewire delivery catheter;
Figure 1B shows the guidewire of Figure 1A in a deployed configuration, with the guidewire delivery catheter partially withdrawn and the anchor radially expanded;
Figure 2 shows a schematic illustration of a prior art guidewire deployed in the pulmonary artery;
Figure 3 shows the prior art guidewire of Figure 2 in the pulmonary artery with a delivered percutaneous pulmonary valve on a covered stent in the correct position;
Figure 4 shows a guidewire according to an embodiment of the invention deployed in the pulmonary trunk;
Figure 5 shows a guidewire according to an embodiment of the invention deployed into the left pulmonary artery;
Figure 6 shows the invention deployed in a distal sub-branch of the left pulmonary artery;
Figure 7 shows how, with the anchor of an embodiment of the present invention deployed, pushing on the guidewire can be used to cause it to bend radially outwards;
Figure 8 shows how, with the anchor of an embodiment of the present invention deployed, pulling on the guidewire can be used to cause it to bend radially inwards.
Figure 9A shows a front view of a guidewire according to an alternative embodiment of the invention;
Figure 9B shows a side view of the guidewire of Figure 9A;
   Figure 10A is a schematic illustration of a guidewire according to an embodiment of the present invention deployed within a body cavity, duct or vessel to which a small longitudinal pulling force has been applied;
Figure 10B shows a schematic illustration of the guidewire embodiment of Figure 10A to which a larger pulling force has been applied;
Figure 10C shows a schematic illustration of the guidewire embodiment of Figure 10B to which a still larger pulling force has been applied, causing buckling of the anchor portion;
Figure 10D shows a schematic illustration of the guidewire embodiment of Figure 10C to which a still larger pulling force has been applied, causing reversal of the guidewire profile and removal of the guidewire from the body cavity, duct or vessel;
Figure 11 shows a schematic sectional front view in the region of box A in Figure 10A showing the profile of the portion of the anchor in contact with the wall of the body cavity, duct or vessel;
Figures 12A to 12C depicts a process of deploying an anchor comprising a reversibly expandable frame;
Figures 13A to 13C depicts a process of retracting the anchor of Figures 12A to 12C;
Figures 14A to 14C depict an anchor according to an alternative embodient comprising a reversibly expandable frame;
Figure 15A depicts a guidewire within a straight delivery catheter;
Figure 15B depicts a guidewire in a bent delivery catheter, showing how frictional forces inhibit insertion through curved pathways;
Figure 16A depicts a guidewire contained within a cylindrical spacer, within a delivery catheter;
Figure 16B shows a guidewire within a delivery catheter, with a low friction coating applied to both the guidewire and inner surface of the delivery catheter;
Figure 17A shows an anchor having two friction reducing elements in the expanded (deployed) state;
Figure 17B shows the anchor of Figure 17B in the radially contracted state, ready for insertion;
Figures 17C is a perspective view of the anchor of Figure 17A;
Figure 18 is a schematic side view of an anchor having a leading edge region of lower stiffness to reduce the risk of tissue trauma; and
Figures 19A and 19B are schematic side and end views respectively of an anchor having a leading edge region which is angled radially inwards to reduce the risk of tissue trauma.

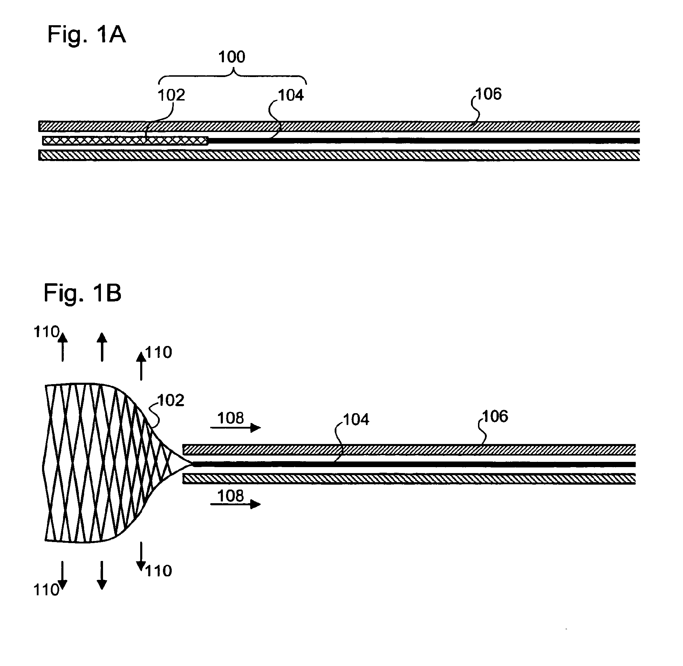

Figures 1A and 1B show an example of a guidewire 100 comprising a stem 104 and an anchor portion 102. In Figure 1A, the guidewire is fully contained within a guidewire delivery catheter 106, which may be tubular in form, for example. The anchor 102 is radially constrained by the catheter 106, which may be desirable during an insertion phase to reduce the risk of injury or discomfort to a patient.

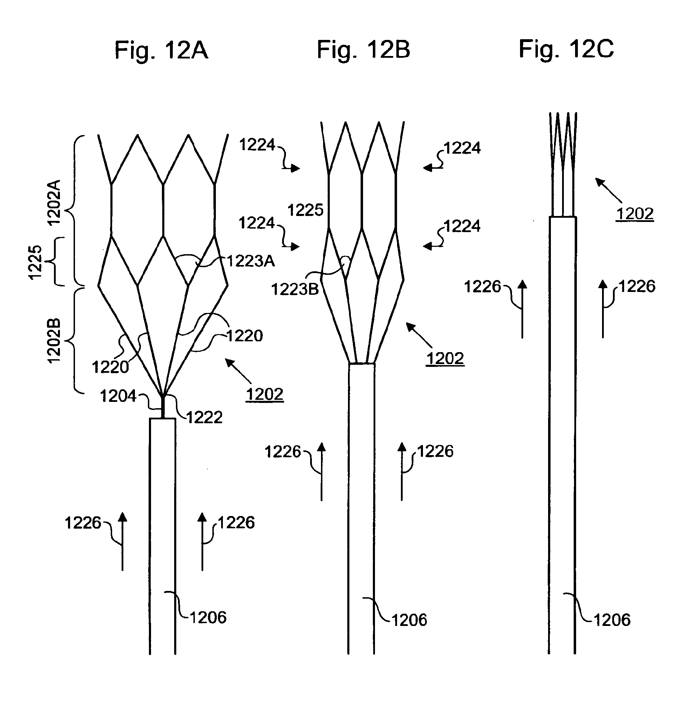

Once the guidewire is fully deployed within the body cavity, duct or vessel, the guidewire delivery catheter 106 can be longitudinally withdrawn (arrows 108) so as to expose the anchor 102. A guidewire 100 and catheter 106 in this configuration are shown in Figure 1B. Being no longer radially constrained, the anchor 102 springs open (arrows 110) and can perform its function of anchoring the guidewire 100. The anchor 102 may be reversibly expandable, optionally through a plurality of cycles of expansion and retraction. This allows the anchor 102 to be removed easily and/or to be re-positioned (where a plurality of expansion/retraction cycles are possible). Further details of embodiments which rely on longitudinal movement of the delivery catheter to control radial expansion of an anchor can be found below with reference to Figures 12a to 14.

Other deployment mechanisms may also be adopted without departing from the scope of the invention. For example, a mechanism which does not rely on longitudinal movement of the catheter may be provided for initiating transformation of the anchor from a radially constrained (narrow) configuration suitable for insertion of the guidewire to a radially expanded state suitable for performing the anchoring function. Additionally or alternatively, the anchor 102 may be configured so that the guidewire 100 can be inserted while the anchor 102 is in an expanded state, so that no transformation from a radially constrained to an expanded state is required. For example, the anchor 102 may be configured so that it provides only a limited force in opposition to insertion of the guidewire 100 while applying a significantly greater force in opposition to applied pulling forces (opposite to the insertion direction). In such a configuration, the guidewire 100 can be inserted relatively easily, but can nevertheless provide a substantial anchoring force against pulling of the guidewire 100 out of the body cavity, duct or vessel, without a separate mechanism being required for switching between a radially constrained and radially expanded state.

The anchor 102 according to this embodiment is configured so that when it is deployed against the wall of the body cavity, duct or vessel, there exists a line of contact between the outer surface of the anchor 102 and the wall that takes the form of a closed path. In the example shown, there are variety of such closed paths. For example, the borders of the closed cells ("closed" because their borders are continuous) of the lattice making up the anchor 102 constitute closed paths. These are examples of closed paths which may be described as being axially perpendicular to the axis of the guidewire in the region of deployment (i.e. they face outwards against the wall of the tissue against which the anchor is to press and provide a gripping force). Alternatively, it is also possible to trace a zig-zag path around the circumference of the anchor 102, forming a closed loop that is axially parallel to the guidewire 104 in the region of deployment (i.e. the loop is aligned with the axis of the lumen, for example the blood vessel). In both cases, as the anchor 102 is pressed against the tissue, tissue is forced to protrude in between the gaps in the anchor material (either side of the closed path lines of contact) and resists longitudinal movement of the anchor 102 relative to the tissue.

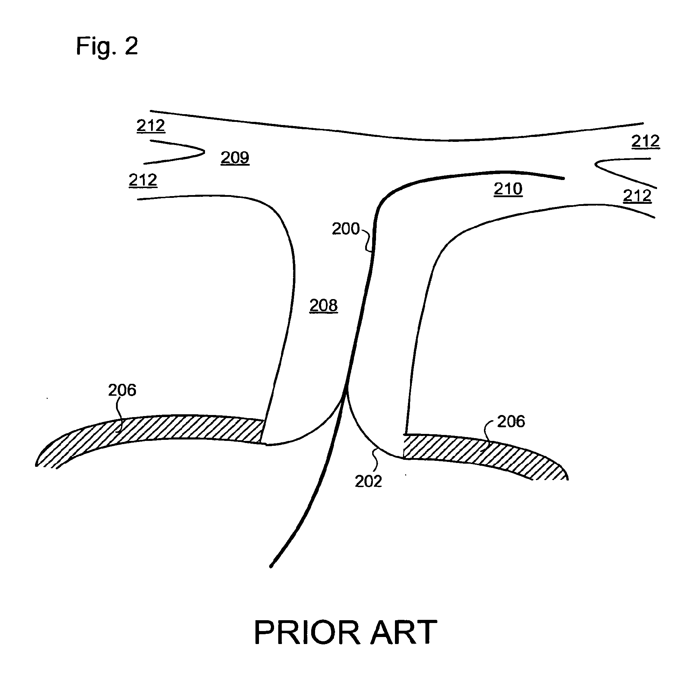

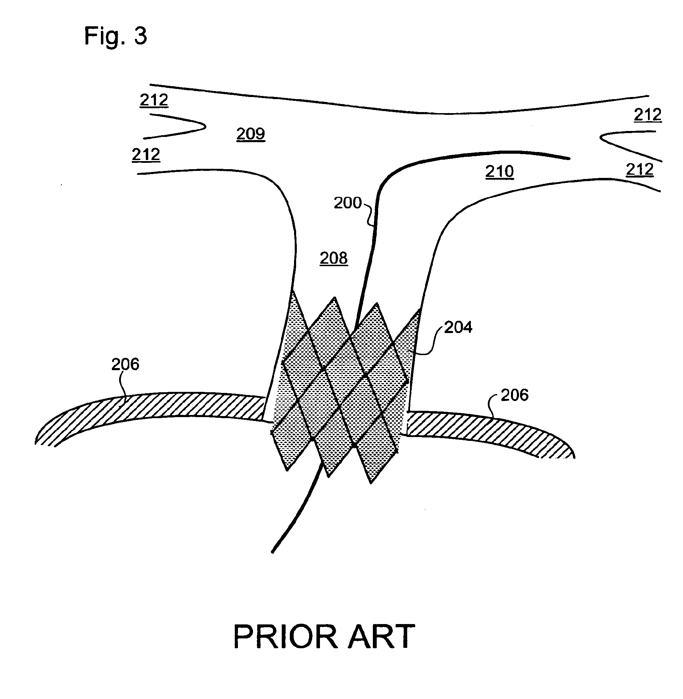

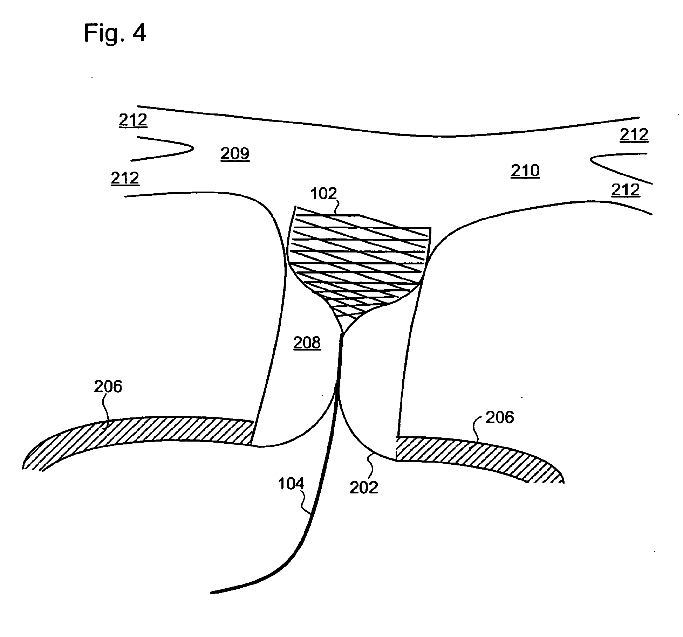

Figures 2 to 8 show a schematic of a portion of the pulmonary artery system and right ventricle. In particular, a portion of the pulmonary trunk 208 is shown passing through the right ventricle wall 206 and extending into a left and right pulmonary artery 209 and 210 and distal sub-branches 212. Figures 2 to 8 show various configurations of fully deployed guidewires: Figures 2 and 3 show prior art arrangements; and Figures 4 to 8 illustrate application of embodiments of the present invention.

Figures 2 and 3 illustrate snapshots in a procedure for inserting a covered stent 204 into the pulmonary trunk 208. Figure 2 shows a preparatory step wherein only the guidewire 200 is inserted into the pulmonary trunk 208, with a free end of the guidewire 200 extending into the left pulmonary artery 210. Once the guidewire 200 is in place, various delivery catheters can be brought to the treatment site via the guidewire 200 in order to carry out the required intervention. In the present example, the result of the intervention is the placement of a covered stent 204 as shown in Figure 3.

Where the anatomy is tortuous and/or where small bend radii are present, considerable amounts of stress may need to be applied to the delivery catheter and/or the guidewire. Frequently, it is necessary to pull and push the guidewire 200 and catheter relative to each other in order to try to straighten out the delivery catheter and make it easier to push it over the guidewire 200. This relative movement between the delivery catheter and guidewire may be difficult to control because the end of the guidewire is free. Furthermore, such manipulation may lead to significant longitudinal displacement of the guidewire, which may risk injury to the patient. For example, the free end of the guidewire may puncture interior walls of the body cavity, duct or vessel due to excessive longitudinal displacement of the guidewire 200.

The provision of the anchor 102 according to embodiments of the present invention can significantly mitigate these problems. The anchor 102 inhibits longitudinal displacement of the guidewire 100, which makes it easier to control the precise position of the guidewire in the body cavity, duct or vessel. This improved control may apply either or both to the longitudinal position of the guidewire 100 and the radial position of the guidewire at the site of interest. The longitudinal position of the guidewire 100 may be controlled both because the anchor 102 prevents excessive movement in the longitudinal direction by counteracting pulling and/or pushing forces applied to the guidewire 100, but also because the anchor 102 may be configured to preferentially anchor at openings and/or junctions in the body cavity, duct or vessel. For example, the anchor 102 may be configured to expand to a diameter which is slightly greater than a tubular body cavity, duct or vessel within which the guidewire 100 is deployed so as to block the end of the guidewire 100 at the position corresponding to an opening of the tube into a larger volume. Alternatively or additionally, the anchor 102 may be arranged so that, when deployed, the part of the guidewire stem 104 immediately adjacent to the anchor 102 is brought towards the middle of the body cavity, duct or vessel (pulmonary trunk in Figure 4, for example) within which the end part of the guidewire 100 extends. Alternatively, the anchor 102 may be configured to position the part of the guidewire stem 104 immediately adjacent to the anchor 102 at other positions within the cross section of the body cavity, duct or vessel within which the end part of the guidewire 100 is located (i.e. at positions radially displaced from the centre of the body cavity, duct or vessel).

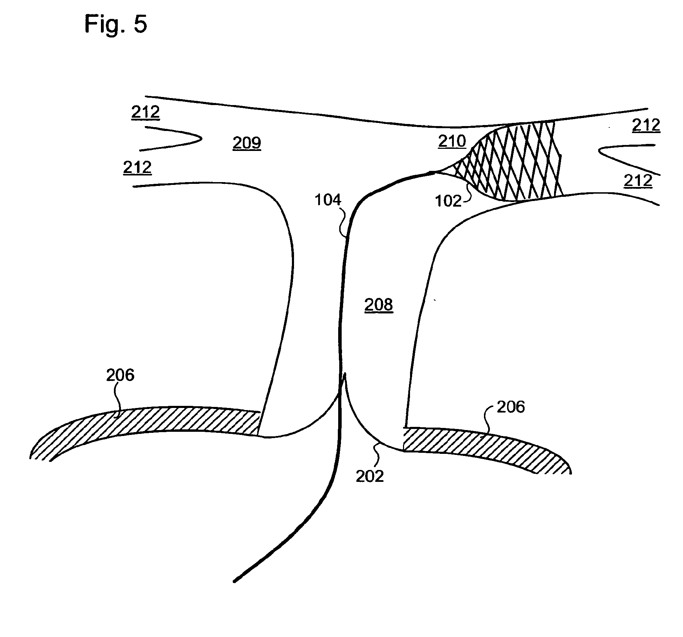

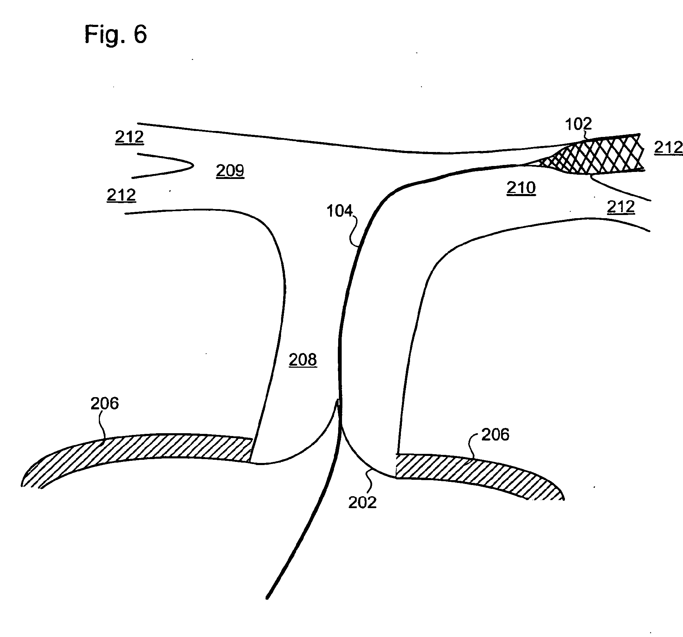

In the context of guidewires for pulmonary valve replacement, the anchor 102 may be configured to engage at various positions within the pulmonary vascular system. Figures 4 to 6 show three possibilities: Figure 4 shows the anchor 102 engaging in the pulmonary trunk, Figure 5 shows the anchor 102 engaging in the left pulmonary artery 210, and Figure 6 shows the anchor 102 engaging in a distal sub-branch 212 of the left pulmonary artery 210. The anchor 102 may be configured so that it can engage effectively in cavities of different radial sizes, for example to engage in each of the positions shown in Figures 4 to 6, without modification of the anchor 102 (i.e. with the same anchor). Alternatively, the anchor may be formed so as to be more specialist, working significantly more efficiently in cavities having cross sectional areas of a particular size or size range. In this case, it might be beneficial to provide different anchors 102 for each of the three different applications shown in Figures 4 to 6. The properties of the anchor 102 can be tuned by varying the shape or material of the anchor, or, where the anchor is formed from shaped wire, by varying the thickness of the wire.

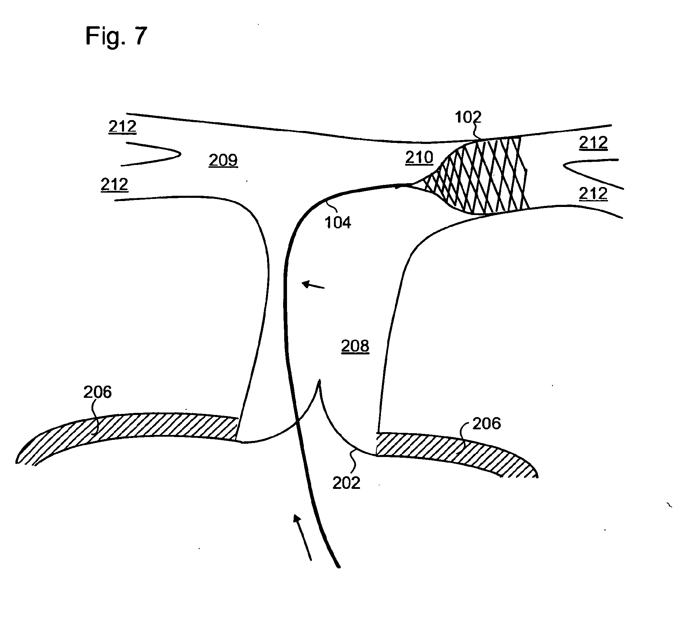

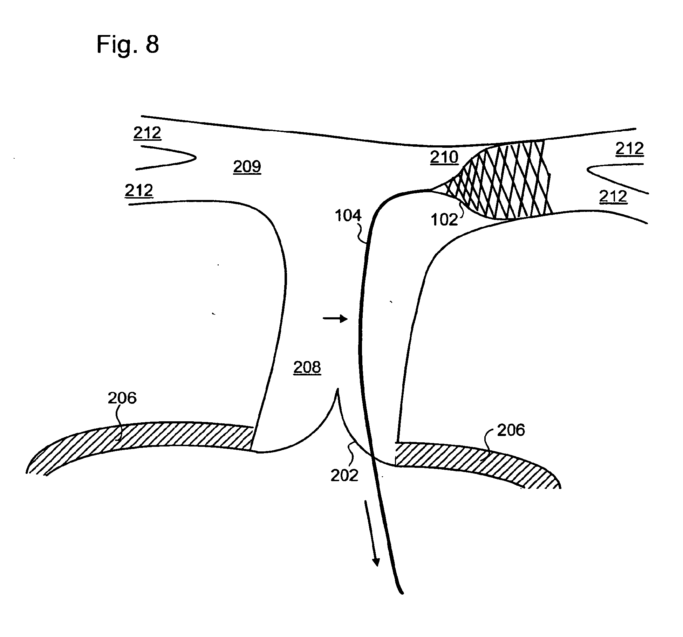

As discussed above, one of the advantages of the guidewire 100 of the present invention is that the anchor 102 makes it possible to apply a greater longitudinal pulling and/or pushing force without the end of the guidewire 100 undergoing a corresponding longitudinal displacement forwards or backwards. In addition to reducing the risk of injury and loss of position of the guidewire end in the body cavity, duct or vessel, the fact that the end of the guidewire is fixed also facilitates greater manipulation of the form of the guidewire stem 104 in the region between the anchor 102 and the user and outside world. In particular, as shown in Figures 7 and 8, pushing and pulling of the anchored guidewire 100 can significantly and controllably alter the form of the guidewire stem 104 within the body cavity, duct or vessel. Figure 7 illustrates how a pushing force (i.e. a force applied in a direction parallel to an insertion direction) can be effective to increase a curvature of the guidewire stem 104 in certain regions of its path (in the example of Figure7, in the pulmonary trunk) and/or to push the guidewire stem 104 towards a given wall of the body cavity, duct or vessel (in the example of Figure 7, towards the right wall of the pulmonary trunk). Figure 8 illustrates how pulling on the guidewire 100 can lead to a corresponding reduction in the curvature of the guidewire stem 104 and a pulling of the guidewire stem 104 towards an opposing wall of the body cavity, duct or vessel (in the example of Figure 8, the left wall of the pulmonary trunk). These kinds of manipulation can be used greatly to facilitate insertion of a delivery catheter over the guidewire 100 to a treatment site. In the prior art arrangements, it was only possible to try to straighten out the delivery catheter by pushing and pulling of the guidewire 200 and catheter relative to each other, and this only to a limited extent. The present embodiment makes it possible also to manipulate the shape of the guidewire 100. Because the guidewire is more fixedly positioned within the body, it may also be possible to achieve a greater degree of "straightening out" of the delivery catheter by applying increased and more controlled forces between the guidewire 100 and delivery catheter 106.

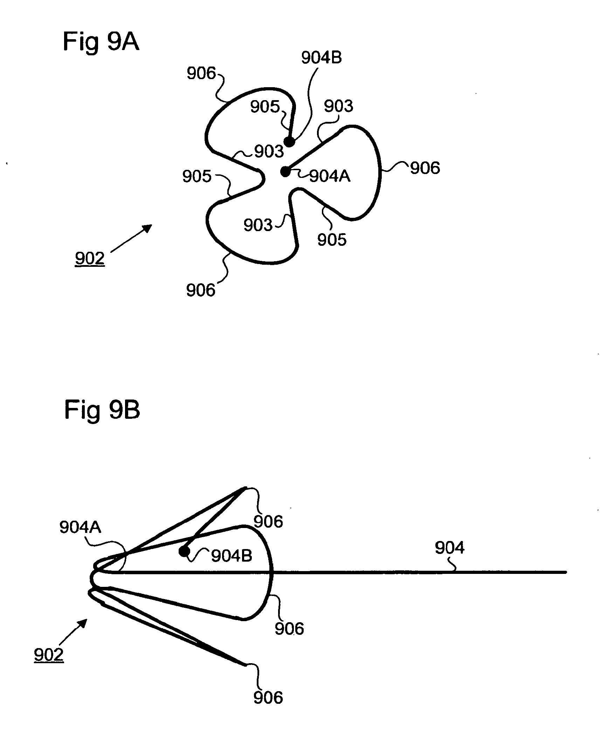

In the embodiments of Figures 1 and 4 to 8, the anchor 102 is configured to be self-expanding and is formed separately from the guidewire stem 104. Other arrangements are, however, possible. For example, the anchor 102 may be configured to expand only on application of an actuating force, which could be applied by various means. Alternatively or additionally, the anchor 102 may be formed integrally with the guidewire stem 104. According to an embodiment, the guidewire stem 104 and anchor 102 may be formed from a single piece of wire, with the anchor 102 constructed by appropriate shaping of the wire at a distal end. Figures 9A and 9B show a configuration of this type.

Figure 9A shows a front view of a guidewire 900 formed from a single piece of wire. According to this embodiment, the anchor 902 is formed by bending the wire from an end 904A of the stem 904 into a form having looped portions 906 bending back generally in a direction having a vector component anti-parallel to an insertion direction in the region of the anchor 902 and a vector component pointing radially outwards relative to an axis defined by the stem 904 of the guidewire 900. Figure 9B is a side view of the embodiment of Figure 9A.

Preferably, the looped portions 906 are formed by shaping the guidewire to follow a first path 903 which at all points has a vector component opposite to an insertion direction of said wire (i.e. to the right in Figure 9B) in the region of said anchor 902 and, subsequently, a second path 905 having at all points a vector component parallel to said insertion direction of said guidewire (i.e. to the left in Figure 9B) in the region of said anchor 902.

Looped shapes of these types are particularly suitable for producing anchoring forces of appropriate sizes with a minimum risk of injury or discomfort.

The looped portions or "petals" 906 may further be configured such that, when a pulling force is applied to the guidewire 900, the shape of curvature tends to push radially outwards towards the walls of the body cavity, duct or vessel within which the anchor 902 is deployed, forming a "lock wire". A rising pulling force on the wire will result in a rising radial force up until a "breakaway" point where the anchor 902 will collapse and/or unravel, and fixation is lost.

The breakaway point may be defined by reference to a threshold force. For all pulling and/or pushing forces below the threshold force, the anchoring force provided by the anchor 902 increases so as to resist longitudinal movement of the guidewire 900 in the direction of the pulling or pushing. If a force is equal to or greater than the threshold force, the anchor 902 collapses and can no longer sustain an anchoring force commensurate with the size of the pulling or pushing force (i.e. the anchoring force irreversibly falls to a value substantially less than the threshold force) and the anchor 902 thus slips.

The breakaway or threshold force can be set appropriately by suitable selection of materials and/or cross-sections to ensure that, in practice, it is higher than the traction forces likely to be required for device delivery.

An advantage of this approach is that, once a particular procedure is completed, the guidewire 900 can either be pulled with a force greater than the threshold force to remove it, or a catheter can be fed over the wire to the first bend (the end 904A of the stem 904) and the wire drawn back straight through the catheter.

Figures 9A and 9B illustrate this principle for a particular shape of anchor. In this embodiment, three looped portions or "petals" 906 act as non-return catches allowing traction to be placed on the wire. To release the wire, it is pulled to the point where the petals fold back on themselves (at the threshold force) and the guidewire 900 is released.

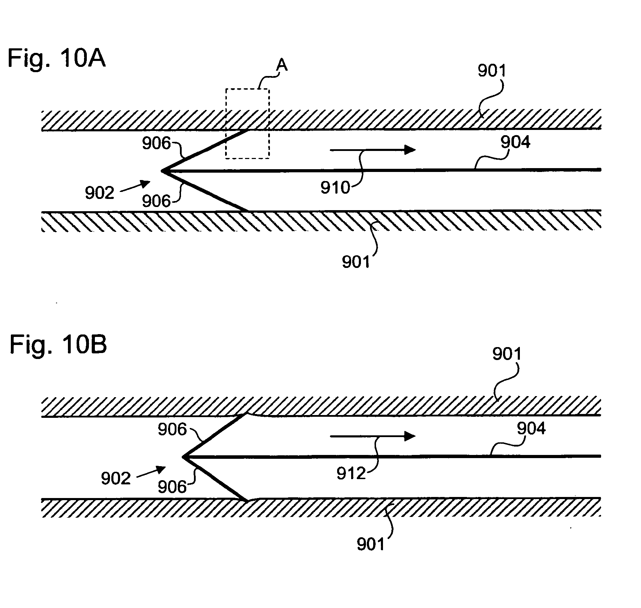

Figures 10A to 10D illustrate this process schematically. In Figure 10A, a relatively small pulling force is applied (arrow 910). The guidewire 900 inserted in vessel 901 is not deformed to any significant extent, and a relatively small radially outward force is applied by the loop members 906 of the anchor 902 against the walls of the vessel 901.

As the pulling force increases (arrow 912), the radially outward force applied by the loop members 906 of the anchor 902 increases, leading to increased local deformation of the walls of the vessel 901 and an increased anchoring force (which balances the pulling force 912).

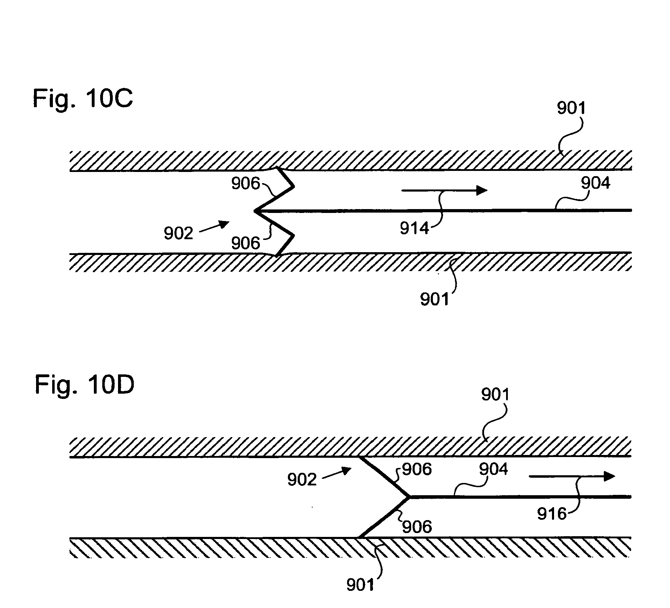

Figure 10C shows the situation shortly after a pulling force (arrow 914) has been applied that exceeds the threshold force, leading to buckling of the loop members 906 of the anchor 902. At this point, the radially outward force applied by the loop members 906 of the anchor 902 and/or the longitudinal anchoring force provided by the anchor 902 greatly diminishes, leading to slip or release of the guidewire 900 from its anchored position.

Figure 10D illustrates the situation after a collapse of the kind illustrated in Figure 10C, with the guidewire 100 being removed by a pulling force which may now be smaller than the threshold force (arrow 916), the buckled anchor 902 no longer being effective to provide any substantial anchoring force. The guidewire can thus be easily removed from the body cavity, duct or vessel with a minimum risk of damage. The relatively large force required to buckle the anchor 902 need only be provided for a short period of time.

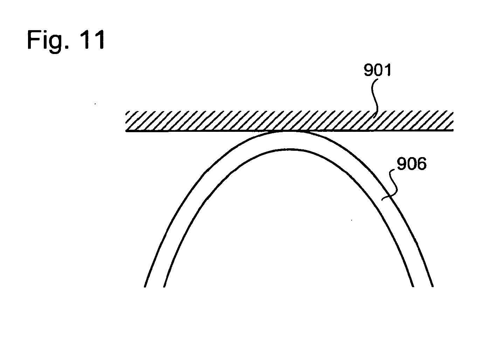

Figure 11 shows schematically how a radially extreme portion of one of the loop members 906 may come into contact with the walls of the vessel 901 in the region delimited by the broken line rectangle A in Figure 10A. According to this embodiment, the looped members 906 are arranged so that only smooth continuous (unbroken) portions of the anchor 902 come into contact with the walls of the vessel 901. More generally, the anchor 906 may be shaped so that, when deployed, an end of the wire is prevented from coming into contact with the walls of the body cavity, duct or vessel by other portions of the anchor 906. By ensuring that the end of the guidewire is kept radially away from the walls of the vessel and/or that only relatively smooth portions of the wire forming the anchor 906 are allowed to come into direct contact with the vessel walls 901, the risk of injury and/or irritation to the patient is reduced.

According to embodiments of the invention, the anchor 102 may be positioned at a distal end of the guidewire 100. However, it is also possible to locate the anchor 102 at intermediate positions of the guidewire 100, so that there is a portion of guidewire stem 104 before and after the anchor 102.

According to embodiments of the invention, the anchor 102 may be configured to provide an anchoring force primarily in an insertion direction (so as to balance a pulling force). However, it is also possible to configure the anchor 102 to provide an anchoring force primarily in a direction opposite to the insertion direction (so as to balance a pushing force). Alternatively, the anchor 102 may be configured to provide anchoring forces in both pulling and pushing directions. The maximum anchoring forces (i.e. threshold forces) may be the same or different in the pulling and pushing directions.

Figures 12A to 14 illustrate an anchor 1202 that is reversibly switchable between a radially constrained (narrow) state to a radially expanded (wide, anchoring) state. In the other words, the anchor 1202 according to this embodiment can be inserted into the body cavity, vessel or duct to be treated in a radially constrained state, expanded to perform its anchoring function when the guidewire is in place, and, at a subsequent time, retracted again when the guidewire is to be removed. In a preferred embodiment, this cycle can be carried out a plurality of times to allow the guidewire to be positioned at a plurality of different locations. This functionality may be useful to tune the position of the guidewire for maximal performance for a given operation or set of operations, using relatively small changes in the position of the anchoring site. Alternatively or additionally, this functionality may assist where operations are to be carried out using substantially different anchoring points, for example because the treatment location is substantially different for different operations or because a variable (controllable) quality of anchoring is desirable. For example, anchoring within certain (for example, relatively narrow or fragile) portions of the body cavity, vessel or duct may, in certain embodiments, provide a relatively strong anchor (i.e. high maximal anchoring forces) or convenient positioning, but may stress the region of tissue in contact with the anchor 1202. It may therefore be beneficial to locate the anchor 1202 in such regions for a minimum length of time and use the reversible expandability to re-position the anchor 1202 in a more robust location for subsequent operations that are less demanding (from the point of view of required anchoring forces and/or positioning convenience).

According to the embodiments of Figures 12A to 14, the switch from the radially constrained state to the radially expanded state and vice versa is actuated by longitudinal movement of a tubular delivery catheter 1206.

Figures 12A to 12C are top plan views of a guidewire delivery catheter 1206, guidewire 1204 and anchor 1202. Longitudinal advance of the catheter 1206 causes the anchor 1202 to collapse radially from an expanded state (Figure 12A) to a partially radially constrained state (Figure 12B) to a fully radially constrained state (Figure 12C).

The anchor 1202 according to this embodiment comprises an engaging portion 1202A, configured in use to engage with the walls of the body cavity, vessel or duct in order to provide an anchoring force. The engaging portion 1202A may be radially self-expanding and/or substantially cylindrical in form, for example.

The anchor 1202 additionally comprises a connecting portion 1202B for connecting the engaging portion 1202A to the stem of the guidewire 1204. The connecting portion 1202B is configured such that longitudinal advancement of the delivery catheter 1206 (see arrows 1226) causes engagement of the catheter 1206 with the connecting portion 1202B with the result that the connected portion 1202B is forced to take a radially more constrained form; this in turn forces the engaging portion 1202A to take a radially more constrained form. For example, as shown in Figures 12A to 12C, the connecting portion 1202B may comprise a plurality of angled rods 1220, which diverge obliquely (relative to the axis of the anchor) from the root 1222 of the anchor 1202 so as to connect to the engaging portion 1202A at a plurality of circumferentially separated (different) points at a longitudinally proximal end of the engaging portion 1202A. Longitudinal advancement of the catheter 1206 forces the angle that the angled rods 1220 make with respect to the axis of the anchor 1202 to decrease, which in turn decreases the separation of the distal ends of the rods 1220 from the axis of the anchor 1202 as well as reducing the circumferential separation of the distal ends of the rods 1220. The engaging portion 1202A is formed from a frame that can react to such changes in the positions of the distal ends of the rods 1220 by adopting a suitably radially constrained form. This contraction process is illustrated in Figure 12B (see arrows 1224). The anchor 1220 may extend by a small amount during the radial contraction as elements within the frame become more aligned with each other. In the present embodiment, the engaging portion 1202A remains substantially cylindrical during radial contraction (e.g. through the state depicted in Figure 12B) and after complete radial contraction has taken place (Figure 12C). However, other configurations are also possible, as long as the radial extent of the engaging portion and/or the radial and/or maximal anchoring force decreases as the catheter 1206 advances against the connecting portion 1202B.

The sub-rods in the axial zone 1225 immediately after the rods 1220 form a closed loop zig-zag pattern around the axis of the anchor 1202, defining in use a closed path or loop which will be in contact with the tissue with which grip is desired. As the anchor 1202 switches from the radially expanded state to the radially constrained state, the angle 1223A between adjacent sub-rods (or "elements" of the contact line between the engaging portion of the anchor and the tissue) decreases. This may also be described as a decrease in the average angle between the sub-rods and a direction (lying within the cylindrical surface of the lumen within which the anchor is deployed) parallel to the axis of the anchor (or guidewire or lumen). This effect tends to decrease the gripping force provided by the anchor (as desired), in addition to the reduction achieved by reducing the radially outward force provided by the fact that the anchor is contracting.

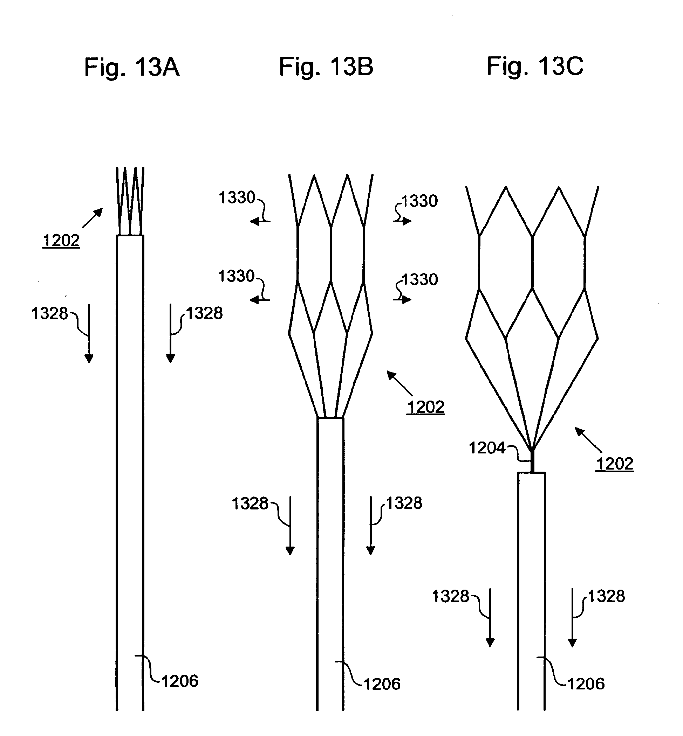

Figures 13A to 13C shows the reverse sequence, with a retracting catheter 1206 (see arrows 1328) allowing radial expansion of the anchor 1202 (see arrows 1330). This radial expansion may be driven by self-expanding properties of the connecting portion 1202B and/or of the engaging portion 1202A. In any event, retraction of the catheter 1206 causes the angle of the rods 1220 to increase, which in turn causes the frame of the engaging portion 1202A to expand radially.

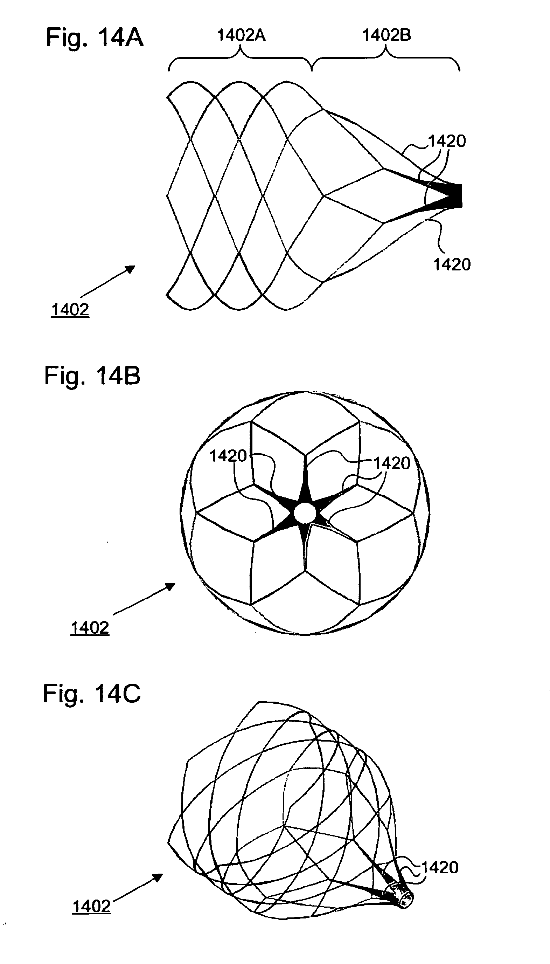

Figures 14A to 14C show an alternative embodiment of an anchor 1402 that is configured to operate according to the same general principle described above with reference to Figures 12A to 13C. Figure 14A is a side view; Figure 14B is an end view; and Figure 14C is a perspective view.

Longitudinal advancement of a catheter (not shown) will press against diverging angled rods 1420 of a connecting portion 1402B to cause radial contraction of a substantially cylindrical engaging portion 1402A in a similar manner to the embodiment of Figures 12A to 13C. However, whereas in the embodiment of Figures 12A to 13C the angled rods 1220 were formed from substantially the same material (same thickness and composition) as the rods making up the frame of the engaging portion 1202A, in the present embodiment, the angled rods 1420 are reinforced relative to the rods making up the frame of the engaging portion 1402A. This reinforcement may be achieved by increasing the thickness of the angled rods 1420 (as shown schematically in Figures 14A to 14C) and/or by changing the material of the angled rods 1420, for example.

The role of the angled rods 1420 in providing the necessary elastic forces for the transition from the radially contracted state to the radially expanded state may be increased if these elements are reinforced. According to a particular variation, the angled rods 1420 may be configured to provide substantially all of the force necessary for the transition. In this way, the properties of the engaging portion 1402A can be focussed on the task of engaging effectively with the walls of the body cavity, vessel or duct (i.e. so as to provide the necessary frictional forces with a minimum of irritation to the patient).

Reinforcement of the connecting portion 1202B/1402A may also be advantageous from the point of view of robustness to repeated cycles of expansion and contraction, for example where a significant amount of tuning and/or re-positioning of the anchor 1202/1402 is necessary.

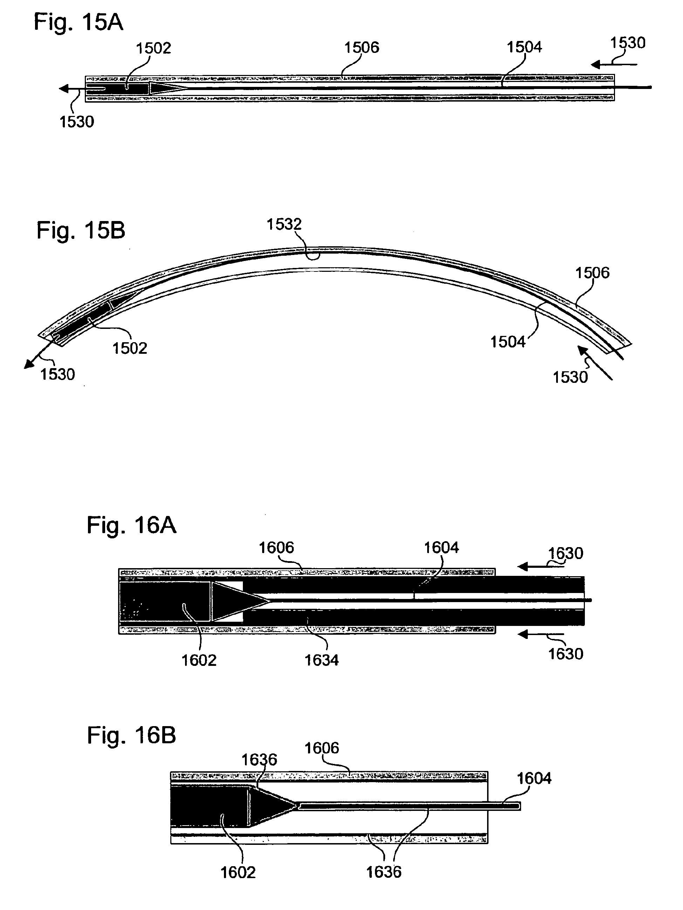

Figures 15A and 15B illustrate a difficulty that can be encountered when inserting an anchor and guidewire into position at the site of interest by applying a longitudinal insertion force 1530.

Where the delivery catheter 1506 is straight (Figure 15A) the anchor 1502 and guidewire 1504 can pass through relatively easily. However, it will generally be the case that the path through the patient to the site where the anchor 1502 will be deployed will involve a number of relatively sharp bends. In this case, as shown in Figure 15B, the guidewire 1504 will tend to be pushed radially outwards against an outer inner surface 1532 of the catheter 1506, which leads to a large frictional force resisting insertion of the anchor 1502 and guidewire 1504.

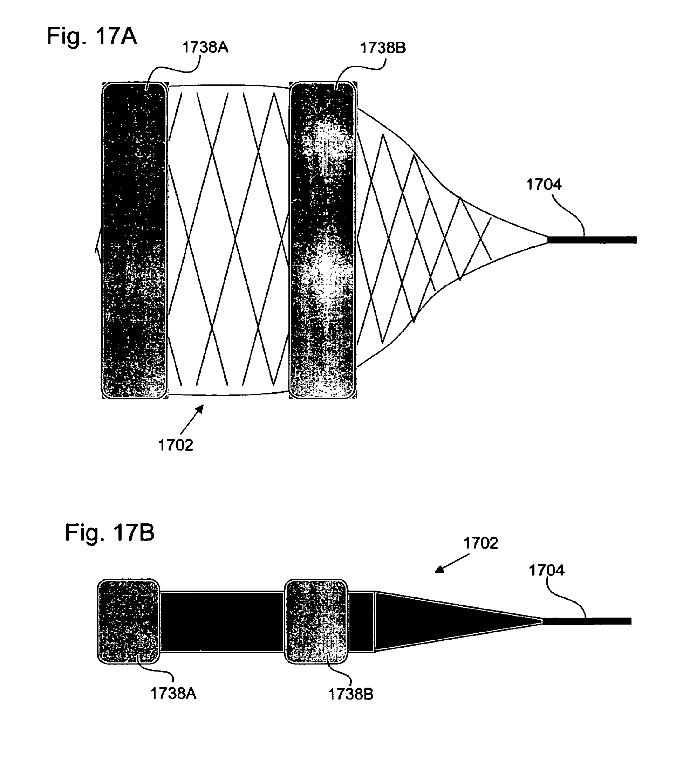

Figures 16A, 16B, and 17A to 17D depict alternative approaches for addressing this problem.

In Figure 16A, a hollow cylindrical spacer 1634 is provided (pushed in the direction of arrows 1630) behind the anchor 1602 and between the inner surface of the delivery catheter 1606 and the guidewire 1604. This cylindrical spacer 1634 constrains the guidewire 1604 laterally to stay within a cylindrical region axially centered within the catheter 1606 and having a cross-sectional area that is less than 80 percent, preferably less than 50 percent, and more preferably less than 10 percent, of the total cross-sectional area of the catheter 1606. Constraining the guidewire 1604 in this way reduces the size of frictional forces generated when the guidewire 1604 is forced through bends (at least partly by reducing the angle at which the guidewire 1604 is forced into contact with the inner surface of the constraining element, i.e. spacer or catheter).

Figure 16B shows an alternative arrangement in which the inner surface of the catheter 1606 is coated with a low friction material 1636 (alternatively, the low friction material may be integral with the catheter 1606). Optionally, all or a portion of the outer surface of the anchor 1602 or all or a portion of the guidewire 1604 may also be coated or formed from a low friction material 1636.

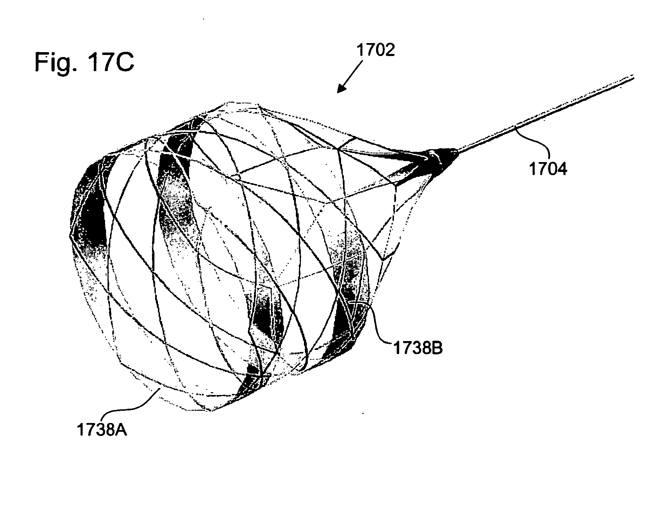

Figures 17A to 17C illustrate an alternative arrangement in which low friction sections or "bearings" 1738A/B are attached to the anchor 1702. Figure 17A shows the anchor 1702 in its deployed expanded state, while Figure 17B shows the anchor 1702 in the radially constrained state, for insertion. Figure 17C is a perspective view of the anchor 1702 of Figure 17A.

In this case, the low friction bearings 1738A/B are formed from tubes of a low friction polymer. Two bearings are provided in the example shown, one 1738A near a leading edge and the other 1738B near the trailing edge of the anchor 1702.

The bearings 1738A/B provided a low friction contact between the anchor 1702 and the inner walls of the delivery catheter that assists insertion of the anchor 1702 and guidewire 1704 through the delivery catheter when the anchor 1702 is in a radially constrained state. Preferably, the bearings 1738A/B are configured so as to protrude radially further than the gripping part of the anchor 1702 in the radially constrained state (as shown in Figure 17B), so that the bearings 1738A/B press against the inner wall of the delivery catheter in preference to the anchor. Preferably, the bearings 1738A/B protrude to such an extent that there is no contact between the anchor 1702 and the delivery catheter during insertion, even where the delivery path is curved (up to a threshold curvature).

When the anchor 1702 is deployed in use, the bearings 1738A/B may still come into contact with the walls of the body cavity, duct or vessel with which grip is to be made (although this is not obligatory), but they will not prevent the anchor 1702 from performing its function.

Where the bearings 1738A/B do not cover all of the engaging portion of the anchor 1702, there will be regions of the engaging portion that will press against the walls of the tissue and provide a gripping force in the normal way.

Where the bearings (or a low friction coating) covers all of the engaging portion of the anchor 1702, grip will still be achieved because the radially outward force of the anchor 1702 against the walls of the tissue will still cause the tissue to protrude inwards relative to the anchor and provide a grip, particularly where the engaging portion is configured to form a line of contact along a closed path, for example as closed cells pressing against the walls or as an axially aligned loop.

The low friction coatings and/or materials discussed above may be formed from a variety of materials having the desired properties. In general, the static dry coefficient of friction should be lower than 0.5, preferably lower than 0.1 and more preferably lower than 0.05. For example, PTFE could be used.

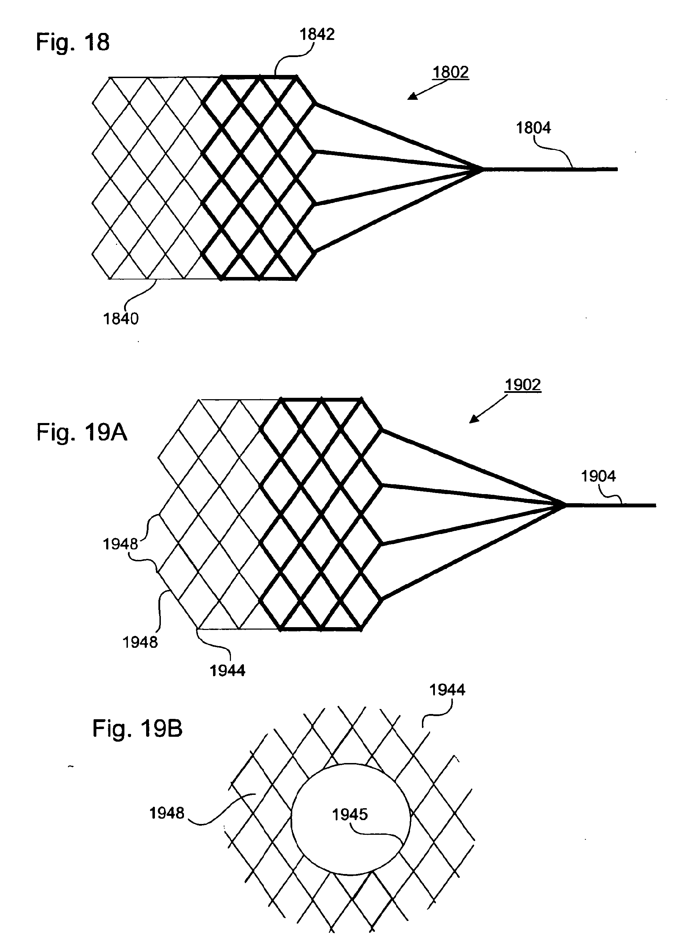

Figure 18 shows an anchor 1802 having leading edge region 1840 of lower stiffness (achieved using a material of lower thickness and/or lower stiffness) relative to the adjacent region 1842. The leading edge of such an anchor 1802 yields more easily and the risk of trauma to tissue is reduced.

Figures 19A and 19B show an alternative arrangement for reducing the risk of tissue trauma caused by the leading edge 1945 of the anchor 1902. Figure 19A is a side-view of the arrangement; Figure 19B is a front view. Here, a portion 1948 of the leading edge region is arranged to dip radially inwards from axial point 1944 forwards to the leading edge 1945, away from the tissue wall (in use). The region immediately behind point 1944 presses against the tissue wall and provides the support necessary to maintain the separation between the radially inward portion 1948 and the tissue wall. In Figures 19A and 19B, the anchor comprises a region of lower stiffness and a region of higher stiffness in the manner of the arrangement of Figure 18, to further reduce the risk of tissue trauma, but this is optional: the arrangement may be provided separately if desired.

## Claims

1. A guidewire (100) for a catheter, comprising:
an anchor (102) for providing an anchoring force that inhibits longitudinal movement of the guidewire (100) when the guidewire (100) is deployed within a duct or vessel; wherein:
said anchor (102) is configured to press radially outwards in order to grip said duct or vessel and thereby provide said anchoring force, **characterized in that**:
said anchor (102) comprises an engaging portion configured to engage with said duct or vessel when said anchor (102) is deployed in use such that there is a line of contact between said engaging portion and said duct or vessel in the form of a closed path enclosing a region where there is no contact between said engaging portion and said duct or vessel.

2. A guidewire (100) according to claim 1, wherein said anchor (102) is configured such that said closed path spans a cross-sectional portion of said duct or vessel,
said closed path optionally forming a loop having an axis that is aligned with a longitudinal axis of said duct or vessel in the location where the anchor (102) is deployed.

3. A guidewire (100) according to claim 1, wherein said anchor (102) is configured such that said closed path presses against one lateral side only of said duct or vessel.

4. A guidewire (100) according to claim 3, wherein said anchor (102) is configured such that said closed path forms a loop having an axis that is aligned perpendicularly to a longitudinal axis of said duct or vessel in the location where the anchor (102) is deployed.

5. A guidewire (100) according to any one of claims 1 to 4, wherein said anchor (102) can be reversibly switched between a radially constrained state suitable for insertion of the guidewire (100) into said duct or vessel and a radially expanded state suitable for providing said anchoring force, wherein, optionally:
an average angle between elements of said closed path and a direction parallel to the longitudinal axis of said anchor (102) is larger in the radially expanded state than in the radially constrained state, the guidewire (100) being optionally further configured such that longitudinal movement of a delivery catheter can cause said anchor (102) to switch between said radially constrained state and said radially expanded state.

6. A guidewire (1204) according to claim 5, wherein said anchor (1202) further comprises a connecting portion (1202B) for connecting said engaging portion (1202A) to a stem of said guidewire (1204), wherein said connecting portion (1202B) is configured such that longitudinal advancement of a delivery catheter (1206) so as to engage with said connecting portion (1202B) can cause switching of said anchor (1202) between said radially expanded state and said radially constrained state,
wherein said engaging portion (1202A) optionally comprises a collapsible frame or collapsible latticework having closed cells defined by said closed paths, and
wherein said connecting portion (1202B) optionally comprises a plurality of rods (1220) obliquely angled relative to the axis of said anchor (1202) and diverging from a root thereof to a plurality of different points of connection with said engaging portion (1202A).

7. A guidewire (100) according to any one of the preceding claims, further comprising:
a friction-reducing element provided on a radially outer portion of said anchor (102) to facilitate insertion of said anchor (102) into position within said duct or vessel,
wherein optionally said friction-reducing element is formed from a material having a static dry coefficient of friction below 0.1, and is arranged so as to be positioned in use between at least a portion of said anchor (102) and a wall of said duct or vessel, in contact with said wall of said duct or vessel.

8. A guidewire according to any one of the preceding claims, wherein said anchor (902) is configured to provide an anchoring force when deployed that is substantially equal to an externally applied force up to a predetermined threshold force, wherein:
said anchor (902) is further configured such that said anchoring force falls to a value substantially less than said threshold force after an external force is applied that is equal to or greater than said threshold force;
said guidewire is formed from an elongated wire and said anchor (902) is formed by shaping a portion of said wire so as to provide said anchoring force when deployed within a duct or vessel;
said portion of said wire is shaped so as to form an at least partial loop (906), contact between said anchor (902) and said wall of said duct or vessel being made via an unbroken part of said loop (906), to provide said anchoring force;
said loop is configured to collapse for applied forces equal to or greater than said predetermined force, so as to exert an anchoring force substantially less than said threshold force or substantially no anchoring force; and
said loop (906) is formed by shaping the guidewire to follow a first path which at all points has a vector component opposite to an insertion direction of said wire in the region of said anchor (902) and, subsequently, a second path having at all points a vector component parallel to said insertion direction of said guidewire in the region of said anchor (902).

9. A guidewire according to claim 8, wherein said portion of wire is shaped so as to form a plurality of said loops (906).

10. A guidewire according to any one of the preceding claims wherein said anchor (902) is formed by shaping a portion of wire and is positioned at an end of said guidewire, and said anchor (902) is shaped so that, when deployed, an end of said wire is prevented from coming into contact with the walls of said duct or vessel by the presence of other, more radially outwardly, portions of said anchor (902).

11. A guidewire (100) according to any one of the preceding claims, wherein a portion of said anchor (102) at an axially leading edge is formed so as to have a lower stiffness than an axially adjacent portion of said anchor (102) that is arranged to be in contact with a wall of said duct or vessel when deployed in use.

12. A deployment system, comprising:
a guidewire (1204) according to any one of claims 1 to 11; and
a guidewire delivery catheter (1206) for delivering said guidewire (1204) to a desired location within said duct or vessel, said guidewire delivery catheter (1206) comprising means for actuating said anchor (1202).

13. A deployment system according to claim 12, wherein said means for actuating said anchor (1202) comprises means for selectively switching said anchor (1202) from a radially constrained state to a radially expanded state.

14. A deployment system according to claim 12 or 13, wherein said guidewire delivery catheter (1206) is provided with a hollow cylindrical spacer, to be positioned over said guidewire (1204) and axially behind said anchor (1202), said spacer being configured to laterally constrain the guidewire (1204) within a cylindrical region axially centered within said catheter (1206) and having a cross-sectional area that is less than 50 percent of the total cross-sectional area of the catheter (1206).

15. A deployment system according to any one of claims 12 to 14, wherein at least one of said guidewire (1204), the internal surface of said delivery catheter (1206) or cylindrical spacer, and said anchor (1202) are coated with a low friction coating, said low friction coating providing a static dry coefficient of friction of less than 0.1.

## Patentansprüche

1. Führungsdraht (100) für einen Katheter, umfassend:
eine Verankerung (102), um eine Verankerungskraft bereitzustellen, welche eine Längsbewegung des Führungsdrahts (100) verhindert, wenn der Führungsdraht (100) in einem Kanal oder einem Gefäß eingesetzt ist; wobei:
die Verankerung (102) ausgestaltet ist, um radial nach außen zu drücken, um den Kanal oder das Gefäß zu greifen, und um dadurch die Verankerungskraft bereitzustellen, **dadurch gekennzeichnet:**
**dass** die Verankerung (102) einen Eingriffsabschnitt umfasst, welcher ausgestaltet ist, um sich mit dem Kanal oder mit dem Gefäß in Eingriff zu befinden, wenn die Verankerung (102) eingesetzt wird, so dass eine Kontaktlinie zwischen dem Eingriffsabschnitt und dem Kanal oder dem Gefäß in der Form eines geschlossenen Pfades vorhanden ist, welcher einen Bereich einschließt, in welchem kein Kontakt zwischen dem Eingriffsabschnitt und dem Kanal oder dem Gefäß vorhanden ist.

2. Führungsdraht (100) nach Anspruch 1, **dadurch gekennzeichnet, dass** die Verankerung (102) ausgestaltet ist, so dass der geschlossene Pfad einen Querschnittabschnitt des Kanals oder des Gefäßes überspannt,
wobei der geschlossene Pfad optional eine Schlaufe ausbildet, welche eine Achse aufweist, die mit einer Längsachse des Kanals oder des Gefäßes an der Stelle, an welcher die Verankerung (102) eingesetzt wird, ausgerichtet ist.

3. Führungsdraht (100) nach Anspruch 1, **dadurch gekennzeichnet, dass** die Verankerung (102) ausgestaltet ist, so dass der geschlossene Pfad gegen eine Seite nur des Kanals oder des Gefäßes drückt.

4. Führungsdraht (100) nach Anspruch 3, **dadurch gekennzeichnet, dass** die Verankerung (102) ausgestaltet ist, so dass der geschlossene Pfad eine Schleife ausbildet, welche eine Achse aufweist, die senkrecht zu einer Längsachse des Kanals oder des Gefäßes an der Stelle, an welcher die Verankerung (102) eingesetzt wird, ausgerichtet ist.

5. Führungsdraht (100) nach einem der Ansprüche 1 bis 4, **dadurch gekennzeichnet, dass** die Verankerung (102) umkehrbar zwischen einem radial zusammengepressten Zustand, welcher zur Einführung des Führungsdrahts (100) in den Kanal oder in das Gefäß geeignet ist, und einem radial aufgeweiteten Zustand, welcher geeignet ist, um die Verankerungskraft bereitzustellen, geschaltet werden kann, wobei optional:
ein mittlerer Winkel zwischen Elementen des geschlossenen Pfades und einer Richtung parallel zu der Längsachse der Verankerung (102) in dem radial aufgeweiteten Zustand größer ist als in dem radial zusammengepressten Zustand, wobei der Führungsdraht (100) optional darüber hinaus ausgestaltet ist, so dass eine Längsbewegung eines Einführkatheters bewirken kann, dass die Verankerung (102) zwischen dem radial zusammengepressten Zustand und dem radial aufgeweiteten Zustand geschaltet wird.

6. Führungsdraht (1204) nach Anspruch 5, **dadurch gekennzeichnet, dass** die Verankerung (1202) darüber hinaus einen Verbindungsabschnitt (1202B) umfasst, um den Eingriffsabschnitt (1202A) mit einer Basis des Führungsdrahts (1204) zu verbinden, wobei der Verbindungsabschnitt (1202B) ausgestaltet ist, so dass ein Vorwärtsschieben eines Einführkatheters (1206) in Längsrichtung, um so mit dem Verbindungsabschnitt (1202B) in Eingriff zu kommen, bewirken kann, dass die Verankerung (1202) zwischen dem radial aufgeweiteten Zustand und dem radial zusammengepressten Zustand geschaltet wird,
wobei der Eingriffsabschnitt (1202A) optional einen zusammenlegbaren Rahmen oder ein zusammenlegbares Gitternetz mit geschlossenen Zellen, welche durch die geschlossenen Pfade definiert sind, umfasst, und
wobei der Verbindungsabschnitt (1202B) optional mehrere Streben (1220) umfasst, welche schräg relativ zu der Achse der Verankerung (1202) abgewinkelt sind und von einer Wurzel davon zu mehreren verschiedenen Verbindungspunkten mit dem Eingriffsabschnitt (1202A) verzweigen.

7. Führungsdraht (100) nach einem der vorhergehenden Ansprüche, darüber hinaus umfassend:
ein eine Reibung reduzierendes Element, welches auf einem radial äußeren Abschnitt der Verankerung (102) vorhanden ist, um eine Einführung der Verankerung (102) in eine Position in dem Kanal oder in dem Gefäß zu ermöglichen,
wobei das die Reibung reduzierende Element optional aus einem Material ausgebildet ist, welches einen statischen Trockenreibkoeffizienten unterhalb von 0,1 aufweist, und ausgestaltet ist, um im Einsatz zwischen zumindest einem Abschnitt der Verankerung (102) und einer Wand des Kanals oder des Gefäßes in Kontakt mit der Wand des Kanals oder des Gefäßes angeordnet zu werden.

8. Führungsdraht nach einem der vorhergehenden Ansprüche, **dadurch gekennzeichnet, dass** die Verankerung (902) ausgestaltet ist, um eine Verankerungskraft bereitzustellen, wenn sie eingesetzt wird, welche im Wesentlichen bis zu einem vorbestimmten Kraftschwellenwert gleich einer von außen aufgebrachten Kraft ist, wobei:
die Verankerung (902) darüber hinaus ausgestaltet ist, so dass die Verankerungskraft auf einen Wert abfällt, welcher im Wesentlichen geringer als der Kraftschwellenwert ist, nachdem eine äußere Kraft aufgebracht wird, welche gleich oder größer als der Kraftschwellenwert ist;
wobei der Führungsdraht aus einem länglichen Draht ausgebildet ist und die Verankerung (902) ausgebildet ist, indem ein Abschnitt des Drahtes geformt wird, um so die Verankerungskraft bereitzustellen, wenn sie in einem Kanal oder in einem Gefäß eingesetzt wird;
wobei der Abschnitt des Drahtes geformt ist, um zumindest eine Teilschleife (906) auszubilden, um einen Kontakt zwischen der Verankerung (902) und der Wand des Kanals oder des Gefäßes bereitzustellen, welche aus einem unzerbrechlichen Teil der Schleife (906) hergestellt ist, um die Verankerungskraft bereitzustellen;
wobei die Schleife ausgestaltet ist, um bei aufgebrachten Kräften, welche gleich oder größer als die vorbestimmte Kraft sind, zusammengelegt zu werden, um so eine Verankerungskraft, welche im Wesentlichen kleiner als der Kraftschwellenwert ist, oder um im Wesentlichen keine Verankerungskraft auszuüben;
wobei die Schleife (906) ausgebildet ist, indem der Führungsdraht geformt wird, um einem ersten Pfad zu folgen, welcher bei allen Punkten eine Vektorkomponente aufweist, welche einer Einführungsrichtung des Drahtes in dem Bereich der Verankerung (902) entgegengerichtet ist, und um anschließend einem zweiten Pfad zu folgen, welcher bei allen Punkten eine Vektorkomponente aufweist, welche parallel zu der Einführungsrichtung des Führungsdrahts in dem Bereich der Verankerung (902) liegt.

9. Führungsdraht nach Anspruch 8, **dadurch gekennzeichnet, dass** der Abschnitt des Drahtes geformt ist, um mehrere Schleifen (906) auszubilden.

10. Führungsdraht nach einem der vorhergehenden Ansprüche, **dadurch gekennzeichnet, dass** die Verankerung (902) durch Formen eines Abschnitts des Drahtes ausgebildet und an einem Ende des Führungsdrahts angeordnet ist, und dass die Verankerung (902) geformt ist, so dass, wenn sie eingesetzt wird, durch das Vorhandensein von anderen, mehr radial außen liegenden, Abschnitten der Verankerung (902) verhindert wird, dass ein Ende des Drahtes in einen Kontakt mit den Wänden des Kanals oder des Gefäßes kommt.

11. Führungsdraht (100) nach einem der vorhergehenden Ansprüche, **dadurch gekennzeichnet, dass** ein Abschnitt der Verankerung (102) an einer axial außen liegenden Kante ausgebildet ist, um eine geringere Steifigkeit als ein axial innen liegender Abschnitt der Verankerung (102) aufzuweisen, welcher ausgestaltet ist, um sich mit einer Wand des Kanals oder des Gefäßes, wenn sie eingesetzt wird, in Kontakt zu befinden.

12. Einsatzsystem umfassend:
einen Führungsdraht (1204) nach einem der Ansprüche 1 bis 11; und
einen Führungsdraht-Einführkatheter (1206), um den Führungsdraht (1204) an eine gewünschte Stelle innerhalb des Kanals oder des Gefäßes zu bringen, wobei der Führungsdraht-Einführkatheter (1206) Mittel zur Betätigung der Verankerung (1202) umfasst.

13. Einsatzsystem nach Anspruch 12, **dadurch gekennzeichnet, dass** die Mittel zur Betätigung der Verankerung (1202) Mittel umfassen, um selektiv die Verankerung (1202) aus einem radial zusammengepressten Zustand in einen radial aufgeweiteten Zustand zu schalten.

14. Einsatzsystem nach Anspruch 12 oder 13, **dadurch gekennzeichnet, dass** der Führungsdraht-Einführkatheter (1206) mit einem hohlen zylindrischen Abstandshalter bereitgestellt ist, welcher über dem Führungsdraht (1204) und axial hinter der Verankerung (1202) angeordnet ist, wobei der Abstandshalter ausgestaltet ist, um den Führungsdraht (1204) seitlich in einem zylindrischen Bereich axial zentriert innerhalb des Katheters (1206) zu halten, und eine Querschnittsfläche aufweist, welche kleiner als 50 % der gesamten Querschnittsfläche des Katheters (1206) ist.

15. Einsatzsystem nach einem der Ansprüche 12 bis 14, **dadurch gekennzeichnet, dass** zumindest der Führungsdraht (1204), die Innenfläche des Einführkatheters (1206) oder der zylindrische Abstandshalter und die Verankerung (1202) mit einer eine geringere Reibung aufweisenden Beschichtung beschichtet sind, wobei die die geringe Reibung aufweisende Beschichtung einen statischen Trockenreibkoeffizienten von weniger als 0,1 aufweist.

## Revendications

1. Fil de guidage (100) pour cathéter, comprenant :
une ancre (102) pour fournir une force d'ancrage qui empêche le mouvement longitudinal du fil de guidage (100) quand le fil de guidage (100) est déployé à l'intérieur d'un conduit ou d'un vaisseau ; dans lequel :
ladite ancre (102) est configurée pour exercer une pression radialement vers l'extérieur afin de s'accrocher audit conduit ou vaisseau et fournir ainsi ladite force d'ancrage, **caractérisé en ce que** :
ladite ancre (102) comprend une partie de mise en prise configurée pour se mettre en prise avec ledit conduit ou vaisseau quand ladite ancre (102) est déployée en utilisation de telle manière qu'il y a une ligne de contact entre ladite partie de mise en prise et ledit conduit ou vaisseau sous la forme d'un chemin fermé délimitant une région dans laquelle il n'y a pas de contact entre ladite partie de mise en prise et ledit conduit ou vaisseau.

2. Fil de guidage (100) selon la revendication 1, dans lequel ladite ancre (102) est configurée de telle manière que ledit chemin fermé parcourt une partie de section transversale dudit conduit ou vaisseau,
ledit chemin fermé formant éventuellement une boucle ayant un axe qui est aligné avec un axe longitudinal dudit conduit ou vaisseau à l'endroit où l'ancre (102) est déployée.

3. Fil de guidage (100) selon la revendication 1, dans lequel ladite ancre (102) est configurée de telle manière que ledit chemin fermé n'exerce une pression que sur un côté latéral dudit conduit ou vaisseau.

4. Fil de guidage (100) selon la revendication 3, dans lequel ladite ancre (102) est configurée de telle manière que ledit chemin fermé forme une boucle ayant un axe qui est aligné perpendiculairement à un axe longitudinal dudit conduit ou vaisseau à l'endroit où l'ancre (102) est déployée.

5. Fil de guidage (100) selon l'une quelconque des revendications 1 à 4, dans lequel on peut changer l'état de ladite ancre (102) de façon réversible entre un état contraint radialement pour l'insertion du fil de guidage (100) dans ledit conduit ou vaisseau et un état dilaté radialement, approprié pour fournir ladite force d'ancrage, dans lequel, en option :
un angle moyen entre les éléments dudit chemin fermé et une direction parallèle à l'axe longitudinal de ladite ancre (102) est plus grand dans l'état dilaté radialement que dans l'état contraint radialement, le fil de guidage (100) étant en outre éventuellement configuré de telle manière que le mouvement longitudinal d'un cathéter de distribution peut faire changer l'état de ladite ancre (102) entre ledit état contraint radialement et ledit état dilaté radialement.

6. Fil de guidage (1204) selon la revendication 5, dans lequel ladite ancre (1202) comprend en outre une partie de connexion (1202B) pour connecter ladite partie de mise en prise (1202A) à une tige dudit fil de guidage (1204), dans lequel ladite partie de connexion (1202B) est configurée de telle manière que l'avance longitudinale d'un cathéter de distribution (1206) pour le mettre en prise avec ladite partie de connexion (1202B) peut faire changer l'état de ladite ancre (1202) entre ledit état dilaté radialement et ledit état contraint radialement,
dans lequel ladite partie de mise en prise (1202A) comprend en option un cadre pliable ou un treillis pliable ayant des cellules fermées définies par lesdits chemins fermés, et
dans lequel ladite partie de connexion (1202B) comprend en option une pluralité de tiges (1220) formant un angle oblique par rapport à l'axe de ladite ancre (1202) et divergeant depuis un pied de celle-ci, jusqu'à une pluralité de points de connexion différents avec ladite partie de mise en prise (1202A).

7. Fil de guidage (100) selon l'une quelconque des revendications précédentes, comprenant en outre :
un élément réduisant les frottements placé sur une partie radialement extérieure de ladite ancre (102) pour faciliter l'insertion de ladite ancre (102) pour la mettre en position dans ledit conduit ou vaisseau,
dans lequel, en option, ledit élément réduisant les frottements est formé à partir d'un matériau ayant un coefficient de frottement sec statique inférieur à 0,1, et est prévu pour être positionné en utilisation entre au moins une partie de ladite ancre (102) et une paroi dudit conduit ou vaisseau, en contact avec ladite paroi du conduit ou vaisseau.

8. Fil de guidage selon l'une quelconque des revendications précédentes, dans lequel ladite ancre (902) est configurée pour fournir une force d'ancrage quand elle est déployée qui est substantiellement égale à une force appliquée de l'extérieur jusqu'à une force seuil prédéterminée, dans lequel :
ladite ancre (902) est en outre configurée de telle manière que ladite force d'ancrage descend à une valeur sensiblement inférieure à ladite force seuil après application d'une force extérieure qui est supérieure ou égale à ladite force seuil ;
ledit fil de guidage est formé à partir d'un fil métallique allongé et ladite ancre (902) est formée en mettant en forme une partie dudit fil afin de fournir ladite force d'ancrage quand elle est déployée dans le conduit ou vaisseau ;
ladite partie du fil est mise en forme de façon à former une boucle au moins partielle (906), le contact entre ladite ancre (902) et ladite paroi du conduit ou vaisseau étant réalisé par l'intermédiaire d'une partie non brisée de ladite boucle (906), pour fournir ladite force d'ancrage ;
ladite boucle est configurée pour se plier pour des forces appliquées supérieures ou égales à ladite force prédéterminée, afin d'exercer une force d'ancrage sensiblement inférieure à ladite force seuil ou substantiellement pas de force d'ancrage ; et
ladite boucle (906) est formée en mettant en forme le fil de guidage pour suivre un premier chemin qui a en tout point une composante de vecteur opposée à une direction d'insertion dudit fil dans la région de ladite ancre (902), puis un deuxième chemin ayant en tout point une composante de vecteur parallèle à ladite direction d'insertion dudit fil de guidage dans la région de ladite ancre (902).

9. Fil de guidage selon la revendication 8, dans lequel ladite partie de fil est mise en forme de manière à former une pluralité desdites boucles (906).

10. Fil de guidage selon l'une quelconque des revendications précédentes, dans lequel ladite ancre (902) est formée en mettant en forme une partie de fil et est positionnée à une extrémité dudit fil de guidage, et ladite ancre (902) a une forme telle que, quand elle est déployée, une extrémité dudit fil est empêchée de venir au contact des parois dudit conduit ou vaisseau par la présence d'autres parties, plus radialement à l'extérieur, de ladite ancre (902).

11. Fil de guidage (100) selon l'une quelconque des revendications précédentes, dans lequel une partie de ladite ancre (102) est formée, au niveau d'un bord axialement antérieur, de manière à avoir une rigidité inférieure à celle d'une partie axialement adjacente de ladite ancre (102) qui est adaptée pour être en contact avec une paroi dudit conduit ou vaisseau quand elle est déployée.

12. Système de déploiement comprenant :
un fil de guidage (1204) selon l'une quelconque des revendications 1 à 11 ; et
un cathéter de distribution de fil de guidage (1206) pour délivrer ledit fil de guidage (1204) en un endroit souhaité dans ledit conduit ou vaisseau, ledit cathéter de distribution de fil de guidage (1206) comprenant un moyen pour actionner ladite ancre (1202).

13. Système de déploiement selon la revendication 12, dans lequel ledit moyen d'actionnement de ladite ancre (1202) comprend un moyen pour faire passer sélectivement ladite ancre (1202) d'un état contraint radialement à un état dilaté radialement.

14. Système de déploiement selon la revendication 12 ou 13, dans lequel ledit cathéter de distribution de fil de guidage (1206) est pourvu d'une entretoise cylindrique creuse, destinée à être positionnée par dessus ledit fil de guidage (1204) et axialement derrière ladite ancre (1202), ladite entretoise étant configurée pour contraindre latéralement le fil de guidage (1204) dans une région cylindrique centrée axialement à l'intérieur dudit cathéter (1206) et ayant une aire de section transversale qui est inférieure à 50 pour cent de l'aire de section transversale totale du cathéter (1206).

15. Système de déploiement selon l'une quelconque des revendications 12 à 14, dans lequel au mois l'un des éléments suivants : ledit fil de guidage (1204), la surface interne dudit cathéter de distribution (1206) ou de ladite entretoise cylindrique, et ladite ancre (1202) est revêtu d'un revêtement à faible coefficient de frottement, ledit revêtement à faible coefficient de frottement donnant un coefficient de frottement sec statique inférieur à 0,1.
